(19) Europäisches Patentamt
European Patent Office
Office européen des brevets

(11) EP 0 739 878 B2

(12) **NEUE EUROPÄISCHE PATENTSCHRIFT**

(45) Veröffentlichungstag und Bekanntmachung des Hinweises auf die Entscheidung über den Einspruch:
**03.07.2002 Patentblatt 2002/27**

(45) Hinweis auf die Patenterteilung:
**10.06.1998 Patentblatt 1998/24**

(21) Anmeldenummer: **96105509.2**

(22) Anmeldetag: **06.04.1996**

(51) Int Cl.⁷: **C07C 227/08**, C07C 233/36

# (54) Verfahren zur Herstellung hochkonzentrierter fliessfähiger wässriger Lösungen von Betainen

Process for the preparation of concentrated fluid aqueous betaine solutions

Procédé pour la préparation de solutions aqueuses fluides concentrées de bétaine

(84) Benannte Vertragsstaaten:
**BE CH DE ES FR GB IT LI NL**

(30) Priorität: **29.04.1995 DE 19515883**

(43) Veröffentlichungstag der Anmeldung:
**30.10.1996 Patentblatt 1996/44**

(73) Patentinhaber: **Goldschmidt Rewo GmbH & Co. KG**
**36392 Steinau (DE)**

(72) Erfinder:
- **Hamann, Ingo, Dr.**
  **08960 Sant Just, Desvern/Barcelona (ES)**
- **Köhle, Hans-Jürgen, Dr.**
  **36381 Schlüchtern (DE)**
- **Wehner, Winfried, Dr.**
  **36119 Neuhof (DE)**

(56) Entgegenhaltungen:
**EP-A- 0 353 580**
**EP-A- 0 492 228**
**WO-A-95/24376**
**WO-A-95/24377**
**WO-A-96/25389**
**GB-A- 2 022 125**

## Beschreibung

### Beschreibung für folgende Vertragsstaaten : DE, ES, FR, IT

Die Erfindung betrifft ein Verfahren zur Herstellung hochkonzentrierter fließfähiger wäßriger Lösungen von Betainen.

Betaine haben sich in den letzten Jahren in der kosmetischen Industrie als fester Bestandteil von Rezepturen, insbesondere für die Haar- und Körperreinigung, etabliert. Sie haben die Fähigkeit, einen dichten und sahnigen Schaum auszubilden, welcher auch in Gegenwart anderer Tenside, Seifen und Additive über einen langen Zeitraum stabil bleibt, verbunden mit anerkannt guten Reinigungseigenschaften ohne irgendwelche irritierenden Nebenwirkungen, selbst für empfindliche Haut.

Die Herstellung von Betainen wird in der einschlägigen Patent- und Fachliteratur ausführlich beschrieben (US-PS 3 225 074). Im allgemeinen werden dabei tertiäre Aminstickstoffatome enthaltende Verbindungen mit ω-Halogencarbonsäuren oder deren Salze in wäßrigen oder wasserhaltigen Medien umgesetzt. Als tertiäre Aminstickstoffatome enthaltende Verbindungen werden insbesondere Fettsäureamide der allgemeinen Formel (I)

$$R^3\text{-CONH-}(CH_2)_m\text{-}NR^4R^5 \qquad (I)$$

eingesetzt, worin $R^3$ der Alkylrest einer Fettsäure, welcher gegebenenfalls verzweigt, gegebenenfalls Mehrfachbindungen, gegebenenfalls Hydroxylgruppen enthalten kann, $R^4$ und $R^5$ gleich oder verschiedenen Alkylreste mit 1 - 4 C-Atomen bedeuten und m = 1 - 3 sein kann.

Der Alkylrest $R^3$ kann sich hierbei von den natürlichen oder synthetischen Fettsäuren mit 6 - 20 C-Atomen, vorzugsweise von den natürlichen pflanzlichen oder tierischen Fettsäuren mit 8 - 18 C-Atomen ableiten sowie deren natürlich vorkommenden speziell eingestellten Mischungen miteinander oder untereinander.

Als Fettsäuren kommen beispielsweise Capronsäure, Caprylsäure, Caprinsäure, Laurinsäure, Palmitinsäure, Stearinsäure, Linolsäure, Linolensäure, Ricinolsäure in Betracht.

Bevorzugt werden die natürlich vorkommenden Fettsäuremischungen mit einer Kettenlänge von 8 - 18 C-Atomen, wie Kokosfettsäure oder Palmkernfettsäure, welche gegebenenfalls durch geeignete Hydrierungsmethoden gehärtet werden können.

Mit diesen Fettsäuren bzw. Fettsäuremischungen wird durch übliche Kondensationsreaktion bei 140 - 200 °C mit Aminen der allgemeinen Formel (II)

$$H_2N\text{-}(CH_2)_m\text{-}NR^4R^5 \qquad (II)$$

- in welcher $R^4$ und $R^5$ und m die in der Formel (I) genannte Bedeutung haben - zu den Fettsäureamiden mit tertiären Stickstoffatomen der allgemeinen Formel (I) umgesetzt.

Die anschließende Quaternierungsreaktion zu Betainen der Formel (III)

$$R^3\text{-CONH-}(CH_2)_m\text{-}N^+R^4R^5(CH_2)_yCOO^- \qquad (III)$$

worin $R^3$, $R^4$, $R^5$ und m die gleiche Bedeutung wie in den Formeln (I) und (II) haben und y = 1, 2, 3 sein kann, kann nach den literaturbekannten Verfahren durchgeführt werden.

Dem Fettsäureamid der Formel (I) werden dabei in der Regel in wäßrigem Medium ω-Halogenalkylcarbonsäuren, vorzugsweise das Natriumsalz der Chloressigsäure, zugesetzt und bei einer mehrstündigen Reaktion bei ca. 80 - 100 °C die Quaternierung vollzogen. Je nach eingesetzter Fettsäure bzw. Fettsäuremischung muß zum Erhalt der Rührfähigkeit bei fortschreitender Reaktion eine Mindestmenge an Wasser vorhanden sein. Die handelsübliche Konzentration an Betaingehalt der auf diese Weise hergestellten Lösungen liegt daher bei etwa 30 Gew.-% oder darunter.

Zur Einsparung von Lagerungs- und Transportkosten sowie aus formulierungstechnischen Gründen bei der Weiterverarbeitung war aber in vielen Fällen eine höhere Konzentration dringend erwünscht.

In der Vergangenheit sind daher eine Reihe von Verfahren vorgeschlagen worden, welche dieses Problem lösen sollten. So ist aus der DE-PS 3 613 944 ein Verfahren bekannt, bei dem die Quaternierung in einem organischen polaren Lösungsmittel mit einem Wasseranteil von 20 Gew.-% durchgeführt und danach das Lösungsmittel ganz oder teilweise destillativ entfernt und dann wieder mit einem anwendungstechnisch brauchbaren Lösungsmittel auf die gewünschte Konzentration eingestellt wird.

Abgesehen davon, daß das Verfahren technisch aufwendig und kostenintensiv ist, sind organische Lösungsmittel bei der Weiterverarbeitung in kosmetischen Rezepturen vielfach unerwünscht.

Das Verfahren gemäß DE-PS 3 726 322 kommt zwar ohne organische Lösungsmittel aus, jedoch muß die für die Quaternierungsreaktion erforderliche Wassermenge durch Destillation wieder aus dem Reaktionsprodukt entfernt werden, wobei vor oder nach der Einstellung auf die gewünschte Konzentration der pH-Wert der Lösung durch relativ hohe Säuremengen auf hautuntypische Werte von 1 - 4,5 eingestellt werden muß.

Gemäß EP-A-0 353 580 werden dem Reaktionsgemisch aus Fettsäureamid und Halogenalkylcarbonsäure vor oder während der Quaternierungsreaktion oder der erhaltenen Lösung des Betains nichtionogene, wasserlösliche Tenside in solchen Mengen zugesetzt, daß die fertige Lösung 3 - 20 Gew.-% wasserlösliche Tenside enthält.

Als nichtionogene Tenside werden Polyoxyethylenether von Fettsäuren eingesetzt, welche für eine ausreichende Wasserlöslichkeit 10 - 250 Oxyethyleneinheiten enthalten müssen.

Polyoxyethylenether von Fettsäuren mit höheren Anteilen an Oxyethyleneinheiten haben sich hinsichtlich ihrer biologischen Abbaubarkeit aber als nicht unproblematisch erwiesen.

Gemäß der Lehre der WO-A-95/24377 (gültig gemäß Artikel 54(3) EPÜ) werden als Viskositätsregulatoren Mischungen aus a) nichtionischen Tensiden und/oder Hydroxycarbonsäuren und Polyolen und gegebenenfalls freien Fettsäuren bzw. deren Alkalisalze eingesetzt. Gemäß der vorliegenden Erfindung werden weder Polyole noch Fettsäuren mitverwendet.

Gemäß WO-A-95/24376 (gültig gemäß Artikel 54(3) EPÜ) werden als Viskositätsregulatoren Mischungen aus Polyolen (Glycerin) und freien Säuren (Hydroxycarbonsäuren und Fettsäuren) in der Reaktionsmischung eingesetzt. Die Zugabe der Natronlauge erfolgt erst nach Beendigung der Reaktion (Quaternierung) zur Neutralisierung der während der Reaktion freiwerdenden Salzsäure und zur Entfernung von überschüssigen Alkylierungsmitteln.

Gemäß der WO 96/25389 (gültig gemäß Artikel 54(3) EPÜ) soll die kumulierte Aufgabenstellung dadurch gelöst werden, daß man Stickstoffverbindungen als Verflüssigungsmittel vor, während oder nach der Quaternierung zusetzt. Hydroxycarbonsäuren, insbesondere Zitronensäure, werden nicht genannt.

Gemäß EP-A-0 492 228 werden dem Reaktionsgemisch vor oder während der Reaktion 1-10 Gewichts-% anionische Tenside zugesetzt.

Gemäß GB-A-2 022 125 sollen hochkonzentrierte Tensidlösungen erhalten werden durch Vermischen von mindestens zwei Tensiden mit Anteilen von je mindestens 5 %, die eine G-Phase bilden können.

Es bestand daher weiterhin ein Bedarf an hochkonzentrierten, fließ- und pumpfähigen, wäßrigen Lösungen von Betainen, welche frei von niedrigen Alkoholen wie Methanol, Ethanol, Propanol oder Isopropanol oder ionischen und nichtionischen Tensiden sind.

Überraschenderweise wurde nun gefunden, daß man fließ- und pumpfähige, teilweise bis zu ca. 50 Gew.-prozentige Lösungen von Betainen, bezogen auf Trockensubstanz, aus dem Reaktionsgemisch aus Fettsäureamid und ω-Halogenalkylcarbonsäure nach bekannten Verfahren herstellen kann, wenn man vor oder während der Quaternierungsreaktion Alkali- und/oder Erdalkalisalze organischer Säuren, insbesondere mehrbasischer Säuren, wie Citronensäure, zusetzt.

Gegenstand der Erfindung ist daher ein Verfahren zur Herstellung hochkonzentrierter fließ- und pumpfähiger wäßriger Lösungen von Betainen der allgemeinen Formel III

$$R^3\text{-CONH-}(CH_2)_m\text{-}N^+R^4R^5(CH_2)_yCOO^- \qquad \text{(III)}$$

worin $R^3$ der Alkylrest einer Fettsäure, welcher gegebenenfalls verzweigt, gegebenenfalls Mehrfachbindungen, gegebenenfalls Hydroxylgruppen enthalten kann, $R^4$ und $R^5$ gleich oder verschiedenen Alkylreste mit 1 - 4 C-Atomen bedeuten, m = 1 - 3 und y = 1, 2, 3 sein können, durch Quaternierung von tertiären Aminstickstoff enthaltenden Verbindungen der allgemeinen Formel I

$$R^3\text{-CONH-}(CH_2)_m\text{-}NR^4R^5 \qquad \text{(I)}$$

worin $R^3$, $R^4$, $R^5$ und m die obengenannte Bedeutung haben, mit ω-Halogencarbonsäuren nach bekannten Verfahren, welches dadurch gekennzeichnet ist, daß man dem Fettsäureamid der allgemeine Formel I und dem Alkali-/Erdalkalisalz der ω-Halogencarbonsäure vor oder während der Quaternierungsreaktion 0,5 - 5 Gew.-%, bezogen auf Gesamtmischung, mindestens eine der Verbindungen

$$(Li^{1+})_a(X^{n-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

zusetzt, worin $X^{n-}$ der Rest der Zitronensäure mit n = 1 - 3 und im Molekül der Wert von $a^+ = b \cdot n^-$ ist.

Erfindungsgemäß bevorzugt werden Na-citrat, K-citrat, Li-citrat, Mg-citrat, Ca-citrat in Form der wasserfreien Salze oder der Hydrate eingesetzt.

Gemäß EP-A-0 353 580 werden dem Reaktionsgemisch aus Fettsäureamid und Halogenalkylcarbonsäure vor oder während der Quaternierungsreaktion oder der erhaltenen Lösung des Betains nichtionogene, wasserlösliche Tenside in solchen Mengen zugesetzt, daß die fertige Lösung 3 - 20 Gew.-% wasserlösliche Tenside enthält.

Als nichtionogene Tenside werden Polyoxyethylenether von Fettsäuren eingesetzt, welche für eine ausreichende Wasserlöslichkeit 10 - 250 Oxyethyleneinheiten enthalten müssen.

Polyoxyethylenether von Fettsäuren mit höheren Anteilen an Oxyethyleneinheiten haben sich hinsichtlich ihrer biologischen Abbaubarkeit aber als nicht unproblematisch erwiesen.

Gemäß der Lehre der WO-A-95/24377 werden als Viskositätsregulatoren Mischungen aus a) nichtionischen Tensiden und/oder Hydroxycarbonsäuren und Polyolen und gegebenenfalls freien Fettsäuren bzw. deren Alkalisalze eingesetzt. Gemäß der vorliegenden Erfindung werden weder Polyole noch Fettsäuren mitverwendet.

Gemäß WO-A-95/24376, die als Stand der Technik nach Art. 54(3) EPÜ als Stand der Technik für die Vertragsstaaten DE, ES, FR und IT anzusehen ist, werden als Viskositätsregulatoren Mischungen aus Polyolen (Glycerin) und freien Säuren (Hydroxycarbonsäuren und Fettsäuren) in der Reaktionsmischung eingesetzt. Die Zugabe der Natronlauge erfolgt erst nach Beendigung der Reaktion (Quaternierung) zur Neutralisierung der während der Reaktion freiwerdenden Salzsäure und zur Entfernung von überschüssigen Alkylierungsmitteln.

Gemäß der WO 96/25389 soll die kumulierte Aufgabenstellung dadurch gelöst werden, daß man Stickstoffverbindungen als Verflüssigungsmittel vor, während oder nach der Quaternierung zusetzt. Hydroxycarbonsäuren, insbesondere Zitronensäure, werden nicht genannt.

Gesamtaminzahl (GAZ), Tertiäraminzahl (TAZ):

Die Gesamtaminzahl gibt die Anzahl Milligramm Kaliumhydroxid an, die der Gesamtbasizität von 1 Gramm der Aminverbindung äquivalent sind (mg KOH/g).

Die Tertiäraminzahl gibt die Anzahl Milligramm Kaliumhydroxid an, die der Tertiäraminbasizität von 1 Gramm der Aminverbindung äquivalent sind (mg KOH/g).

Die Werte werden bestimmt nach American Oil Chemists Society (A.O.C.S.) Official Method Tf 2a-64.

Chlorid:

Der Gehalt an Chlorid wird potentiometrisch gegen eine Silbernitrat-Maßlösung ermittelt. Als Elektrode wird eine kombinierte Silberchlorid-Elektrode verwendet. Die Werte werden bestimmt nach den Einheitsmethoden der Deutschen Gesellschaft für Fettchemie (DGF): H-III 9.

Beispiele

Beispiel 1:

Herstellung des Aminamids:

98,0 kg gehärtetes Kokosöl wurden in einem Reaktor mit Rührer, Thermometer und Destillationsaufsatz unter Inertgasatmosphäre mit 56,8 kg Dimethylaminopropylamin versetzt und auf 150 - 160 °C erwärmt und unter Rückfluß gekocht. Nach Beendigung der Amidierung (Esterzahl <10 mg KOH/g) wurde deL Aminüberschuß bei dieser Temperatur im Vakuum abdestilliert. Die Destillation war vollständig, als die Differenz aus Gesamtaminzahl und Tertiäraminzahl geringer als 3 mg KOH/g war. Das erhaltene Aminamid hatte eine GAZ von 170,6 mg KOH/g, eine TAZ von 168,6 mg KOH/g und eine Esterzahl von 2,8 mg KOH/g.

Beispiel 2:

97,3 g Monochloressigsäure wurden unter Kühlung in einem 1 l Kolben mit Rührer, Innenthermometer und pH-Elektrode mit 500 g Wasser verdünnt und nach Zugabe von 25,2 g Trinatriumcitrat$\cdot 2H_2O$ mit 85 g 50 %iger wässeriger NaOH-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,3 g Citronensäure-Monohydrat auf 5,4 ein.

Das Endprodukt war eine klare Flüssigkeit mit einer Viskosität von 145 mPas bei 20 °C, einem Trockenrückstand von 45,5 % und einem Chlorid-Gehalt von 3,7 %.

Beispiel 3:

97,3 g Monochloressigsäure wurden unter Kühlung in einem 1 l Kolben mit Rührer, Innenthermometer und pH-Elektrode mit 500 g Wasser verdünnt und nach Zusatz von 11,2 g Trinatriumcitrat·$2H_2O$ mit 85 g 50 %iger wässeriger NaOH-Lösung vorsichtig neutralisiert. Nach Zusatz von 12 g Trimethylglycin und 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 8 h Reaktionszeit war die Alkylierung beendet. Man ließ die Betain-Mischung auf 50 °C abkühlen und stellte den pH-Wert mit 4 g Citronensäure-Monohydrat auf 5,8 ein.

Das Endprodukt war eine klare Flüssigkeit mit einer Viskosität von 140 mPas bei 20 °C, einem Trockenrückstand von 45,2 % und einem Chlorid-Gehalt von 3,5 %.

Beispiel 4:

97,3 g Monochloressigsäure wurden unter Kühlung in einem 1 l Kolben mit Rührer, Innenthermometer und pH-Elektrode mit 500 g Wasser verdünnt, mit 7,2 g Magnesiumcitrat versetzt und mit 35 g Magnesiumhydroxid vorsichtig neutralisiert. Nach Zusatz von 325 g des Aminamids aus Beispiel 1 wurde das Reaktionsgemisch bei 80 - 90°C gerührt. Dabei wurde der pH-Wert zwischen 8 und 8,5 gehalten. Hierzu wurden insgesamt weitere 2,9 g Magnesiumhydroxid benötigt. Nach ca. 8 h Reaktionszeit war die Alkylierung beendet. Man ließ die Betain-Mischung auf 50 °C abkühlen und stellte den pH-Wert mit 6,5 g 50 %iger Citronensäure-Lösung auf 5,1 ein.

Das Endprodukt war eine klare Flüssigkeit mit einer Viskosität von 90 mPas bei 20 °C, einem Trockenrückstand von 45,6 % und einem Chlorid-Gehalt von 3,6 %.

Beispiel 5:

97,3 g Monochloressigsäure wurden unter Kühlung in einem 1 l Kolben mit Rührer, Innenthermometer und pH-Elektrode mit 460 g Wasser verdünnt, mit 17 g Magnesiumcitrat versetzt und mit 38,0 g Magnesiumhydroxid vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt 5,0 g 50 %iger wässeriger NaOH-Lösung benötigt. Nach ca. 8 h Reaktionszeit war die Alkylierung beendet. Man ließ die Betain-Mischung auf 50 °C abkühlen und stellte den pH-Wert mit 4 g Citronensäure-Monohydrat auf 5,7 ein.

Das Endprodukt war eine klare Flüssigkeit mit einer Viskosität von 190 mPas bei 20 °C, einem Trockenrückstand von 49,8 % und einem Chlorid-Gehalt von 3,9 %.

Beispiel 6

97,3 g Monochloressigsäure wurden unter Kühlung in einem 1 l Kolben mit Rührer, Innenthermometer und pH-Elektrode mit 570 g Wasser verdünnt und nach Zugabe von 27,8 g Trikaliumcitrat·$1H_2O$ mit 55,3 g 50 %iger wässeriger KOH-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,0 g Citronensäure-Monohydrat auf 5,4 ein.

Das Endprodukt war eine klare Flüssigkeit mit einer Viskosität von 120 mPas bei 20 °C, einem Trockenrückstand von 45,9 % und einem Chlorid-Gehalt von 3,7 %.

Beispiel 7:

97,3 g Monochloressigsäure wurden unter Kühlung in einem 1 l Kolben mit Rührer, Innenthermometer und pH-Elektrode mit 530 g Wasser verdünnt und nach Zugabe von 24,2 g Trilitiumcitrat·$4H_2O$ mit 23,3 g 50 %iger wässeriger LiOH-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,1 g Citronensäure-Monohydrat auf 5,4 ein.

Das Endprodukt war eine klare Flüssigkeit mit einer Viskosität von 135 mPas bei 20 °C, einem Trockenrückstand von 45,3 % und einem Chlorid-Gehalt von 3,6 %.

Beispiel 8:

97,3 g Monochloressigsäure wurden unter Kühlung in einem 1 l Kolben mit Rührer, Innenthermometer und pH-Elektrode mit 545 g Wasser verdünnt und nach Zugabe von 24,5 g Tricalciumcitrat·4$H_2O$ mit 36,4 g 50 %iger wässeriger $Ca(OH)_2$-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,5 g Citronensäure-Monohydrat auf 5,4 ein.

Das Endprodukt war eine klare Flüssigkeit mit einer Viskosität von 100 mPas bei 20 °C, einem Trockenrückstand von 40,4 % und einem Chlorid-Gehalt von 3,6 %.

**Beschreibung für folgende Vertragsstaaten : BE, CH, LI, GB, NL**

Die Erfindung betrifft ein Verfahren zur Herstellung hochkonzentrierter fließfähiger wäßriger Lösungen von Betainen.

Betaine haben sich in den letzten Jahren in der kosmetischen Industrie als fester Bestandteil von Rezepturen, insbesondere für die Haar- und Körperreinigung, etabliert. Sie haben die Fähigkeit, einen dichten und sahnigen Schaum auszubilden, welcher auch in Gegenwart anderer Tenside, Seifen und Additive über einen langen Zeitraum stabil bleibt, verbunden mit anerkannt guten Reinigungseigenschaften ohne irgendwelche irritierenden Nebenwirkungen, selbst für empfindliche Haut.

Die Herstellung von Betainen wird in der einschlägigen Patent- und Fachliteratur ausführlich beschrieben (US-PS 3 225 074). Im allgemeinen werden dabei tertiäre Aminstickstoffatome enthaltende Verbindungen mit ω-Halogencarbonsäuren oder deren Salze in wäßrigen oder wasserhaltigen Medien umgesetzt. Als tertiäre Aminstickstoffatome enthaltende Verbindungen werden insbesondere Fettsäureamide der allgemeinen Formel (I)

$$R^3\text{-CONH-}(CH_2)_m\text{-}NR^4R^5 \qquad \text{(I)}$$

eingesetzt, worin $R^3$ der Alkylrest einer Fettsäure, welcher gegebenenfalls verzweigt, gegebenenfalls Mehrfachbindungen, gegebenenfalls Hydroxylgruppen enthalten kann, $R^4$ und $R^5$ gleich oder verschiedenen Alkylreste mit 1 - 4 C-Atomen bedeuten und m = 1 - 3 sein kann.

Der Alkylrest $R^3$ kann sich hierbei von den natürlichen oder synthetischen Fettsäuren mit 6 - 20 C-Atomen, vorzugsweise von den natürlichen pflanzlichen oder tierischen Fettsäuren mit 8 - 18 C-Atomen ableiten sowie deren natürlich vorkommenden speziell eingestellten Mischungen miteinander oder untereinander.

Als Fettsäuren kommen beispielsweise Capronsäure, Caprylsäure, Caprinsäure, Laurinsäure, Palmitinsäure, Stearinsäure, Linolsäure, Linolensäure, Ricinolsäure in Betracht.

Bevorzugt werden die natürlich vorkommenden Fettsäuremischungen mit einer Kettenlänge von 8 - 18 C-Atomen, wie Kokosfettsäure oder Palmkernfettsäure, welche gegebenenfalls durch geeignete Hydrierungsmethoden gehärtet werden können.

Mit diesen Fettsäuren bzw. Fettsäuremischungen wird durch übliche Kondensationsreaktion bei 140 - 200 °C mit Aminen der allgemeinen Formel (II)

$$H_2N\text{-}(CH_2)_m\text{-}NR^4R^5 \qquad \text{(II)}$$

- in welcher $R^4$ und $R^5$ und m die in der Formel (I) genannte Bedeutung haben - zu den Fettsäureamiden mit tertiären Stickstoffatomen der allgemeinen Formel (I) umgesetzt.

Die anschließende Quaternierungsreaktion zu Betainen der Formel (III)

$$R^3\text{-CONH-}(CH_2)_m\text{-}N^+R^4R^5(CH_2)_yCOO^- \qquad (III)$$

worin $R^3$, $R^4$, $R^5$ und m die gleiche Bedeutung wie in den Formeln (I) und (II) haben und y = 1, 2, 3 sein kann, kann nach den literaturbekannten Verfahren durchgeführt werden.

Dem Fettsäureamid der Formel (I) werden dabei in der Regel in wäßrigem Medium ω-Halogenalkylcarbonsäuren, vorzugsweise das Natriumsalz der Chloressigsäure, zugesetzt und bei einer mehrstündigen Reaktion bei ca. 80 - 100 °C die Quaternierung vollzogen. Je nach eingesetzter Fettsäure bzw. Fettsäuremischung muß zum Erhalt der Rührfähigkeit bei fortschreitender Reaktion eine Mindestmenge an Wasser vorhanden sein. Die handelsübliche Konzentration an Betaingehalt der auf diese Weise hergestellten Lösungen liegt daher bei etwa 30 Gew.-% oder darunter.

Zur Einsparung von Lagerungs- und Transportkosten sowie aus formulierungstechnischen Gründen bei der Weiterverarbeitung war aber in vielen Fällen eine höhere Konzentration dringend erwünscht.

In der Vergangenheit sind daher eine Reihe von Verfahren vorgeschlagen worden, welche dieses Problem lösen sollten. So ist aus der DE-PS 3 613 944 ein Verfahren bekannt, bei dem die Quaternierung in einem organischen polaren Lösungsmittel mit einem Wasseranteil von 20 Gew.-% durchgeführt und danach das Lösungsmittel ganz oder teilweise destillativ entfernt und dann wieder mit einem anwendungstechnisch brauchbaren Lösungsmittel auf die gewünschte Konzentration eingestellt wird.

Abgesehen davon, daß das Verfahren technisch aufwendig und kostenintensiv ist, sind organische Lösungsmittel bei der Weiterverarbeitung in kosmetischen Rezepturen vielfach unerwünscht.

Das Verfahren gemäß DE-PS 3 726 322 kommt zwar ohne organische Lösungsmittel aus, jedoch muß die für die Quaternierungsreaktion erforderliche Wassermenge durch Destillation wieder aus dem Reaktionsprodukt entfernt werden, wobei vor oder nach der Einstellung auf die gewünschte Konzentration der pH-Wert der Lösung durch relativ hohe Säuremengen auf hautuntypische Werte von 1 - 4,5 eingestellt werden muß.

Gemäß EP-A-0 353 580 werden dem Reaktionsgemisch aus Fettsäureamid und Halogenalkylcarbonsäure vor oder während der Quaternierungsreaktion oder der erhaltenen Lösung des Betains nichtionogene, wasserlösliche Tenside in solchen Mengen zugesetzt, daß die fertige Lösung 3 - 20 Gew.-% wasserlösliche Tenside enthält.

Als nichtionogene Tenside werden Polyoxyethylenether von Fettsäuren eingesetzt, welche für eine ausreichende Wasserlöslichkeit 10 - 250 Oxyethyleneinheiten enthalten müssen.

Polyoxyethylenether von Fettsäuren mit höheren Anteilen an Oxyethyleneinheiten haben sich hinsichtlich ihrer biologischen Abbaubarkeit aber als nicht unproblematisch erwiesen.

Gemäß der Lehre der WO-A-95/24377 (gültig gemäß Artikel 54(3) EPÜ) werden als Viskositätsregulatoren Mischungen aus a) nichtionischen Tensiden und/oder Hydroxycarbonsäuren und Polyolen und gegebenenfalls freien Fettsäuren bzw. deren Alkalisalze eingesetzt. Gemäß der vorliegenden Erfindung werden weder Polyole noch Fettsäuren mitverwendet.

Gemäß WO-A-95/24376 (gültig gemäß Artikel 54(3) EPÜ) werden als Viskositätsregulatoren Mischungen aus Polyolen (Glycerin) und freien Säuren (Hydroxycarbonsäuren und Fettsäuren) in der Reaktionsmischung eingesetzt. Die Zugabe der Natronlauge erfolgt erst nach Beendigung der Reaktion (Quaternierung) zur Neutralisierung der während der Reaktion freiwerdenden Salzsäure und zur Entfernung von überschüssigen Alkylierungsmitteln.

Gemäß der WO 96/25389 (gültig gemäß Artikel 54(3) EPÜ) soll die kumulierte Aufgabenstellung dadurch gelöst werden, daß man Stickstoffverbindungen als Verflüssigungsmittel vor, während oder nach der Quaternierung zusetzt. Hydroxycarbonsäuren, insbesondere Zitronensäure, werden nicht genannt.

Gemäß EP-A-0 492 228 werden dem Reaktionsgemisch vor oder während der Reaktion 1-10 Gewichts-% anionische Tenside zugesetzt.

Gemäß GB-A-2 022 125 sollen hochkonzentrierte Tensidlösungen erhalten werden durch Vermischen von mindestens zwei Tensiden mit Anteilen von je mindestens 5 %, die eine G-Phase bilden können.

Es bestand daher weiterhin ein Bedarf an hochkonzentrierten, fließ- und pumpfähigen, wäßrigen Lösungen von Betainen, welche frei von niedrigen Alkoholen wie Methanol, Ethanol, Propanol oder Isopropanol oder ionischen und nichtionischen Tensiden sind.

Überraschenderweise wurde nun gefunden, daß man fließ- und pumpfähige, teilweise bis zu ca. 50 Gew.-prozentige Lösungen von Betainen, bezogen auf Trockensubstanz, aus dem Reaktionsgemisch aus Fettsäureamid und ω-Halogenalkylcarbonsäure nach bekannten Verfahren herstellen kann, wenn man vor oder während der Quaternierungsreaktion Alkali- und/oder Erdalkalisalze organischer Säuren, insbesondere mehrbasischer Säuren, wie Citronensäure, zusetzt.

Gegenstand der Erfindung ist daher ein Verfahren zur Herstellung hochkonzentrierter fließ- und pumpfähiger wäßriger Lösungen von Betainen der allgemeinen Formel III

$$R^3\text{-CONH-}(CH_2)_m\text{-}N^+R^4R^5(CH_2)_yCOO^- \quad (III)$$

worin $R^3$ der Alkylrest einer Fettsäure, welcher gegebenenfalls verzweigt, gegebenenfalls Mehrfachbindungen, gegebenenfalls Hydroxylgruppen enthalten kann, $R^4$ und $R^5$ gleich oder verschiedenen Alkylreste mit 1 - 4 C-Atomen bedeuten, m = 1 - 3 und y = 1, 2, 3 sein können, durch Quaternierung von tertiären Aminstickstoff enthaltenden Verbindungen der allgemeinen Formel I

$$R^3\text{-CONH-}(CH_2)_m\text{-}NR^4R^5 \quad (I)$$

worin $R^3$, $R^4$, $R^5$ und m die obengenannte Bedeutung haben, mit ω-Halogencarbonsäuren nach bekannten Verfahren, welches dadurch gekennzeichnet ist, daß man dem Fettsäureamid der allgemeine Formel I und dem Alkali-/Erdalkalisalz der ω-Halogencarbonsäure vor oder während der Quaternierungsreaktion 0,5 - 5 Gew.-%, bezogen auf Gesamtmischung, mindestens eine der Verbindungen

$$(Li^{1+})_a(X^{n-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

zusetzt, worin $X^{n-}$ der Rest der Zitronensäure mit n = 1 - 3 und im Molekül der Wert von $a^+$ = b-$n^-$ ist.

Erfindungsgemäß bevorzugt werden Na-citrat, K-citrat, Li-citrat, Mg-citrat, Ca-citrat in Form der wasserfreien Salze oder der Hydrate eingesetzt.

Die Mengen liegen zwischen 0,5 - 5 Gew.-%, bezogen auf Gesamtmischung, vorzugsweise zwischen 1,5 - 2,5 Gew.-%. Die obere Grenze ist nicht kritisch, jedoch sollten zu große Mengen vermieden werden, da sie letztlich den Aktivgehalt vermindern.

Eine weitere Verfahrensvariante besteht darin, daß die $Li^+$-, $Na^+$-, $K^+$-, $Mg^{2+}$-, $Ca^{2+}$-Salze der organischen Säuren ganz oder teilweise - vorzugsweise von 30 - 70 Gew.-% - ersetzt werden durch Verbindungen der allgemeinen Formel (IV)

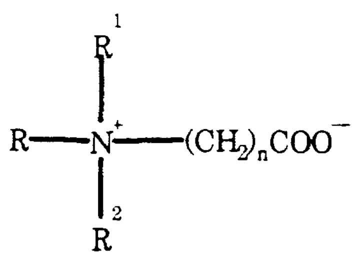

(IV)

worin R, $R^1$, $R^2$ gleich oder verschieden gegebenenfalls verzweigte, gegebenenenfalls Hydroxylgruppen enthaltende Alkylreste mit 1 - 10 C-Atomen, insbesondere Methylreste sind und n = 1 - 3, vorzugsweise n = 1, ist, wobei erfindungsgemäß Mischungen aus 0,5 - 2 Gew.-% Salz und 0,5 - 5 Gew.-% Betain bevorzugt werden.

Erfindungsgemäß mitverwendete Verbindungen der allgemeinen Formel (IV) sind Quaternierungsprodukte von Dimethylethanolamin, Methyldiethanolamin oder Alkyl($C_2$-$C_{10}$)-Dimethylamin und Monochloressigsäure, insbesondere aber das in der Zuckerrübe (Beta vulgaris) natürlich vorkommende Trimethylglycin oder "Betain".

Diese Verbindungen können als Mischungen zusammen mit den Salzen, parallel zur Salzzugabe oder nach der Salzzugabe in Mengen von 1 - 5 Gew.-%, bezogen auf die wäßrige Lösung, der Reaktionsmischung zugesetzt werden, wobei der Gesamtgehalt an Salz + Betain >5 - 7 Gew.-%, bezogen auf die wäßrige Lösung, in der Regel keine Vorteile bringt.

Erfindungsgemäß bevorzugt werden Mischungen aus 0,5 - 2 Gew.-% Salz und 0,5 - 5 Gew.-% Betain.

Die Durchführung des Verfahrens lehnt sich an die im Stand der Technik bekannten Verfahren an, wobei die wesentliche Änderung im Zusatz der Alkali- und/oder Erdalkalisalze der organischen Säuren und gegebenenfalls einer

Verbindung der allgemeinen Formel (IV) vor oder während der Quaternierungsreaktion besteht.

Erfindungsgemäß bevorzugt wird so verfahren, daß die ω-Halogencarbonsäure und die mit mindestens einem Alkali- oder Erdalkalihydroxid neutralisierte Säure vorgelegt werden und während der mehrstündigen Umsetzung in wäßrigem Medium bei 80 - 100 °C der pH-Wert der Lösung durch Zugabe einer Base, vorzugsweise eines Alkalihydroxids, zwischen 8 - 10 gehalten wird und nach der Quaternierung der pH-Wert mit einer organischen Säure, vorzugsweise Citronensäure, auf pH 4,5 - 6 eingestellt wird.

Analysenmethoden

Trockensubstanz:

Die Trockensubstanz wird durch Trocknen des Materials bei 105 °C bis zur Gewichtskonstanz bestimmt. Diese Werte werden bestimmt nach den Einheitsmethoden der Deutschen Gesellschaft für Fettchemie (DGF): B-II.

Säurezahl (SZ):

Die Säurezahl ist ein Maß für die in Fetten und technischen Fettsäuren enthaltenen freien Säuren. Sie gibt die Anzahl Milligramm Kaliumhydroxid an, die notwendig ist, um 1 Gramm Substanz oder technische Fettsäuren zu neutralisieren (mg KOH/g). Diese Werte werden bestimmt nach den Einheitsmethoden der Deutschen Gesellschaft für Fettchemie (DGF): D-IV 2a.

Esterzahl (EZ):

Die Esterzahl ist ein Maß für die in Fetten und technischen Fettsäuren enthaltenen Ester. Sie gibt die Anzahl Milligramm Kaliumhydroxid an, die notwendig ist, um 1 Gramm Substanz oder technische Fettsäuren zu verseifen (mg KOH/g). Diese Werte werden bestimmt nach den Einheitsmethoden der Deutschen Gesellschaft für Fettchemie (DGF): C-V 4.

Gesamtaminzahl (GAZ), Tertiäraminzahl (TAZ):

Die Gesamtaminzahl gibt die Anzahl Milligramm Kaliumhydroxid an, die der Gesamtbasizität von 1 Gramm der Aminverbindung äquivalent sind (mg KOH/g).

Die Tertiäraminzahl gibt die Anzahl Milligramm Kaliumhydroxid an, die der Tertiäraminbasizität von 1 Gramm der Aminverbindung äquivalent sind (mg KOH/g).

Die Werte werden bestimmt nach American Oil Chemists Society (A.O.C.S.) Official Method Tf 2a-64.

Chlorid:

Der Gehalt an Chlorid wird potentiometrisch gegen eine Silbernitrat-Maßlösung ermittelt. Als Elektrode wird eine kombinierte Silberchlorid-Elektrode verwendet. Die Werte werden bestimmt nach den Einheitsmethoden der Deutschen Gesellschaft für Fettchemie (DGF): H-III 9.

Beispiele

Beispiel 1:

Herstellung des Aminamids:

98,0 kg gehärtetes Kokosöl wurden in einem Reaktor mit Rührer, Thermometer und Destillationsaufsatz unter Inertgasatmosphäre mit 56,8 kg Dimethylaminopropylamin versetzt und auf 150 - 160 °C erwärmt und unter Rückfluß gekocht. Nach Beendigung der Amidierung (Esterzahl <10 mg KOH/g) wurde deL Aminüberschuß bei dieser Temperatur im Vakuum abdestilliert. Die Destillation war vollständig, als die Differenz aus Gesamtaminzahl und Tertiäraminzahl geringer als 3 mg KOH/g war. Das erhaltene Aminamid hatte eine GAZ von 170,6 mg KOH/g, eine TAZ von 168,6 mg KOH/g und eine Esterzahl von 2,8 mg KOH/g.

Beispiel 2:

97,3 g Monochloressigsäure wurden unter Kühlung in einem 1 l Kolben mit Rührer, Innenthermometer und

pH-Elektrode mit 500 g Wasser verdünnt und nach Zugabe von 25,2 g Trinatriumcitrat·$2H_2O$ mit 85 g 50 %iger wässeriger NaOH-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,3 g Citronensäure-Monohydrat auf 5,4 ein.

Das Endprodukt war eine klare Flüssigkeit mit einer Viskosität von 145 mPas bei 20 °C, einem Trockenrückstand von 45,5 % und einem Chlorid-Gehalt von 3,7 %.

Beispiel 3:

97,3 g Monochloressigsäure wurden unter Kühlung in einem 1 l Kolben mit Rührer, Innenthermometer und pH-Elektrode mit 500 g Wasser verdünnt und nach Zusatz von 11,2 g Trinatriumcitrat·$2H_2O$ mit 85 g 50 %iger wässeriger NaOH-Lösung vorsichtig neutralisiert. Nach Zusatz von 12 g Trimethylglycin und 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 8 h Reaktionszeit war die Alkylierung beendet. Man ließ die Betain-Mischung auf 50 °C abkühlen und stellte den pH-Wert mit 4 g Citronensäure-Monohydrat auf 5,8 ein.

Das Endprodukt war eine klare Flüssigkeit mit einer Viskosität von 140 mPas bei 20 °C, einem Trockenrückstand von 45,2 % und einem Chlorid-Gehalt von 3,5 %.

Beispiel 4:

97,3 g Monochloressigsäure wurden unter Kühlung in einem 1 l Kolben mit Rührer, Innenthermometer und pH-Elektrode mit 500 g Wasser verdünnt, mit 7,2 g Magnesiumcitrat versetzt und mit 35 g Magnesiumhydroxid vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 2,9 g Magnesiumhydroxid benötigt. Nach ca. 8 h Reaktionszeit war die Alkylierung beendet. Man ließ die Betain-Mischung auf 50 °C abkühlen und stellte den pH-Wert mit 6,5 g 50 %iger Citronensäure-Lösung auf 5,1 ein.

Das Endprodukt war eine klare Flüssigkeit mit einer Viskosität von 90 mPas bei 20 °C, einem Trockenrückstand von 45,6 % und einem Chlorid-Gehalt von 3,6 %.

Beispiel 5:

97,3 g Monochloressigsäure wurden unter Kühlung in einem 1 l Kolben mit Rührer, Innenthermometer und pH-Elektrode mit 460 g Wasser verdünnt, mit 17 g Magnesiumcitrat versetzt und mit 38,0 g Magnesiumhydroxid vorsichtig neutralisiert. Nach Zusatz von 325 g des Aminamids aus Beispiel 1 wurde das Reaktionsgemisch bei 80 - 90°C gerührt. Dabei wurde der pH-Wert zwischen 8 und 8,5 gehalten. Hierzu wurden insgesamt 5,0 g 50 %iger wässeriger NaOH-Lösung benötigt. Nach ca. 8 h Reaktionszeit war die Alkylierung beendet. Man ließ die Betain-Mischung auf 50 °C abkühlen und stellte den pH-Wert mit 4 g Citronensäure-Monohydrat auf 5,7 ein.

Das Endprodukt war eine klare Flüssigkeit mit einer Viskosität von 190 mPas bei 20 °C, einem Trockenrückstand von 49,8 % und einem Chlorid-Gehalt von 3,9 %.

Beispiel 6

97,3 g Monochloressigsäure wurden unter Kühlung in einem 1 l Kolben mit Rührer, Innenthermometer und pH-Elektrode mit 570 g Wasser verdünnt und nach Zugabe von 27,8 g Trikaliumcitrat·$1H_2O$ mit 55,3 g 50 %iger wässeriger KOH-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,0 g Citronensäure-Monohydrat auf 5,4 ein.

Das Endprodukt war eine klare Flüssigkeit mit einer Viskosität von 120 mPas bei 20 °C, einem Trockenrückstand von 45,9 % und einem Chlorid-Gehalt von 3,7 %.

Beispiel 7:

97,3 g Monochloressigsäure wurden unter Kühlung in einem 1 l Kolben mit Rührer, Innenthermometer und

pH-Elektrode mit 530 g Wasser verdünnt und nach Zugabe von 24,2 g Trilitiumcitrat·$4H_2O$ mit 23,3 g 50 %iger wässeriger LiOH-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,1 g Citronensäure-Monohydrat auf 5,4 ein.

Das Endprodukt war eine klare Flüssigkeit mit einer Viskosität von 135 mPas bei 20 °C, einem Trockenrückstand von 45,3 % und einem Chlorid-Gehalt von 3,6 %.

Beispiel 8:

97,3 g Monochloressigsäure wurden unter Kühlung in einem 1 l Kolben mit Rührer, Innenthermometer und pH-Elektrode mit 545 g Wasser verdünnt und nach Zugabe von 24,5 g Tricalciumcitrat·$4H_2O$ mit 36,4 g 50 %iger wässeriger $Ca(OH)_2$-Lösung vorsichtig neutralisiert. Nach Zusatz von 325 g des Aminamids aus Beispiel 1 wurde das Reaktionsgemisch bei 80 - 90 °C gerührt. Dabei wurde der pH-Wert zwischen 8 und 8,5 gehalten. Hierzu wurden insgesamt weitere 5,5 g 50 %iger wässeriger NaOH-Lösung benötigt. Nach ca. 9 h Reaktionszeit war die Alkylierung beendet. Man ließ die Betain-Mischung auf 50 °C abkühlen und stellte den pH-Wert mit 5,5 g Citronensäure-Monohydrat auf 5,4 ein.

Das Endprodukt war eine klare Flüssigkeit mit einer Viskosität von 100 mPas bei 20°C, einem Trockenrückstand von 40,4 % und einem Chlorid-Gehalt von 3,6 %.

## Patentansprüche

### Patentansprüche für folgende Vertragsstaaten : DE, ES, FR, IT

1. Verfahren zur Herstellung hochkonzentrierter fließ- und pumpfähiger wäßriger Lösungen von Betainen der allgemeinen Formel III

$$R^3\text{-CONH-}(CH_2)_m\text{-}N^+R^4R^5(CH_2)_yCOO^- \quad \text{(III)}$$

worin $R^3$ der Alkylrest einer Fettsäure, welcher gegebenenfalls verzweigt, gegebenenfalls Mehrfachbindungen, gegebenenfalls Hydroxylgruppen enthalten kann, $R^4$ und $R^5$ gleich oder verschiedene Alkylreste mit 1 - 4 C-Atomen bedeuten, m = 1-3 und y = 1,2,3 sein können, durch Quaternierung von tertiärem Aminstickstoff enthaltenden Verbindungen der allgemeinen Formel I

$$R^3\text{-CONH-}(CH_2)_m\text{-}NR^4R^5 \quad \text{(I)}$$

worin $R^3$, $R^4$, $R^5$ und m die obengenannte Bedeutung haben, mit ω-Halogencarbonsäuren nach bekannten Verfahren, **dadurch gekennzeichnet, daß** man dem Fettsäureamid der allgemeinen Formel I und dem Alkali-/Erdalkalisalz der ω-Halogencarbonsäure vor oder während der Quaternierungsreaktion 0,5 bis 5 Gew.-%, bezogen auf Gesamtmischung, mindestens eine der Verbindungen

$$(Li^{1+})_a(Xn\text{-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

zusetzt, worin $X^{n-}$ der Rest der Zitronensäure mit n = 1-3 und im Molekül der Wert von $a^+ = b\cdot n^-$ ist.

2. **Verfahren** nach Anspruch 1, **dadurch gekennzeichnet, daß** die in der Lösung enthaltenen Salze zu 30 - 100 Equivalent-% Lithium-, Natrium-, Kalium-, Magnesium- und/oder Calciumsalze sind.

3. Verfahren gemäß Anspruch 1, **dadurch gekennzeichnet, daß** 30-70 Gew.-% der $Li^+$, $Na^+$, $K^+$, $Mg^{2+}$-, $Ca^{2+}$-Salze

mit der organischen Säure ersetzt werden durch Verbindungen der allgemeinen Formel (IV)

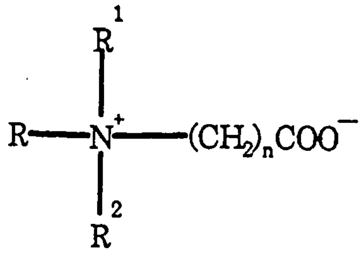

(IV)

worin R, $R^1$, $R^2$ gleich oder verschieden gegebenenfalls verzweigte, gegebenenfalls Hydroxylgruppen enthaltende Alkylreste mit 1 - 10 C-Atomen, insbesondere Methylreste sind und n = 1-3 ist.

**4.** Betainlösungen hergestellt gemäß den Ansprüchen 1 - 3, **dadurch gekennzeichnet, daß** 30 - 70 Gew.-% der $Li^+$, $Na^+$, $K^+$, $Mg^{2+}$-, $Ca^{2+}$-Salze mit der organischen Säure ersetzt werden durch Verbindungen der allgemeinen Formel (IV)

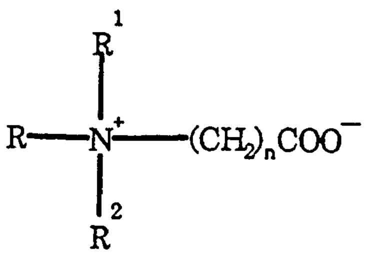

(IV)

worin R, $R^1$, $R^2$ gleich oder verschieden gegebenenfalls verzweigte, gegebenenfalls Hydroxylgruppen enthaltende Alkylreste mit 1 - 10 C-Atomen, insbesondere Methylreste sind und n = 1-3 ist.

**Patentansprüche für folgende Vertragsstaaten : BE, CH, LI, GB, NL**

**1.** Verfahren zur Herstellung hochkonzentrierter fließ- und pumpfähiger wäßriger Lösungen von Betainen der allgemeinen Formel III

$$R^3\text{-CONH-}(CH_2)_m\text{-}N^+R^4R^5(CH_2)_yCOO^- \qquad \text{(III)}$$

worin $R^3$ der Alkylrest einer Fettsäure, welcher gegebenenfalls verzweigt, gegebenenfalls Mehrfachbindungen, gegebenenfalls Hydroxylgruppen enthalten kann, $R^4$ und $R^5$ gleich oder verschiedenen Alkylreste mit 1 - 4 C-Atomen bedeuten, m = 1 - 3 und y = 1, 2, 3 sein können, durch Quaternierung von tertiären Aminstickstoff enthaltenden Verbindungen der allgemeinen Formel I

$$R^3\text{-CONH-}(CH_2)_m\text{-}NR^4R^5 \qquad \text{(I)}$$

worin $R^3$, $R^4$, $R^5$ und m die obengenannte Bedeutung haben, mit ω-Halogencarbonsäuren nach bekannten Verfahren, **dadurch gekennzeichnet, daß** man dem Fettsäureamid der allgemeinen Formel I und dem Alkali-/Erdalkalisalz der ω-Halogencarbonsäure vor oder während der Quaternierungsreaktion 0,5 - 5 Gew.-%, bezogen auf

Gesamtmischung, mindestens eine der Verbindungen

$$(Li^{1+})_a(X^{n-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

zusetzt, worin $X^{n-}$ der Rest der Zitronensäure mit n = 1 - 3 und im Molekül der Wert von $a^+ = b \cdot n^-$ ist.

**2.** Betainlösungen, hergestellt nach Anspruch 1, **dadurch gekennzeichnet, daß** die in der Lösung enthaltenen Salze zu 30 - 100 Equivalent-% Lithium-, Natrium-, Kalium-, Magnesium- und/oder Calciumsalze sind.

**3.** Betainlösungen, hergestellt gemäß den Ansprüchen 1 bis 2, **dadurch gekennzeichnet, daß** sie enthalten

| | |
|---|---|
| >35 Gew.-% | Verbindungen der allgemeinen Formel (III) |
| 1,5 - 3 Gew.-% | mindestens eines Lithium-, Natrium-, Kalium-, Magnesium-, Calciumsalzes |
| ad 100 | Wasser. |

**4.** Verfahren gemäß Anspruch 1, **dadurch gekennzeichnet, daß** 30 - 70 Gew.-% der $Li^+$, $Na^+$, $K^+$, $Mg^{2+}$-, $Ca^{2+}$-Salze der organischen Säure ersetzt werden durch Verbindungen der allgemeinen Formel (IV),

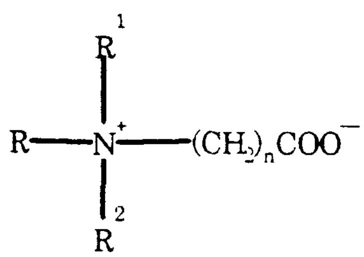

(IV)

worin R, $R^1$, $R^2$ gleich oder verschieden gegebenenfalls verzweigte, gegebenenenfalls Hydroxylgruppen enthaltende Alkylreste mit 1 - 10 C-Atomen, insbesondere Methylreste sind und n = 1 - 3 ist.

**5.** Betainlösungen gemäß den Ansprüchen 2-3 **dadurch gekennzeichnet, daß** 30 - 70 Gew.-% der $Li^+$, $Na^+$, $K^+$, $Mg^{2+}$-, $Ca^{2+}$-Salze der organischen Säure ersetzt werden durch Verbindungen der allgemeinen Formel (IV),

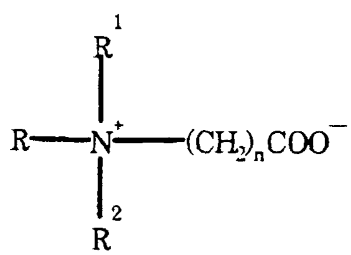

(IV)

worin R, $R^1$, $R^2$ gleich oder verschieden gegebenenfalls verzweigte, gegebenenenfalls Hydroxylgruppen enthaltende Alkylreste mit 1 - 10 C-Atomen, insbesondere Methylreste sind und n = 1 - 3 ist.

## Claims

**Claims for the following Contracting States : DE, ES, FR, IT**

1. Process for preparing highly concentrated flowable and pumpable aqueous solutions of betaines of the general formula III

$$R^3\text{-CONH-}(CH_2)_m\text{-}N^+R^4R^5(CH_2)_yCOO^- \qquad \text{(III)}$$

where $R^3$ is the alkyl radical of a fatty acid, which may be branched, have multiple bonds and contain hydroxyl groups, $R^4$ and $R^5$ are identical or different alkyl radicals having 1 to 4 carbon atoms, m can be 1-3 and y can be 1, 2 or 3, by quaternization of compounds containing tertiary amine nitrogen, of the general formula I

$$R^3\text{-CONH- }(CH_2)_m\text{-}NR^4R^5 \qquad \text{(I)}$$

where $R^3$, $R^4$, $R^5$ and m are as defined above, with ω-halocarboxylic acids by known processes, **characterized in that** before or during the quaternization reaction, 0.5 to 5% by weight, based on the total mixture, of at least one of the compounds

$$(Li^{1+})_a(X^{n-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

where $X^{n-}$ is the radical of citric acid and n = 1-3, and in the molecule the value of $a^+ = b \cdot n^-$, are added to the fatty acid amide of the general formula I and to the alkali metal/alkaline earth metal salt of the ω-halocarboxylic acid.

2. Process according to Claim 1, **characterized in that** the salts present in the solution are 30 - 100 equivalent-% of salts of lithium, sodium, potassium, magnesium and/or calcium.

3. Process according to Claim 1, **characterized in that** 30-70% by weight of the $Li^+$, $Na^+$, $K^+$, $Mg^{2+}$, $Ca^{2+}$ salts with the organic acid are replaced by compounds of the general formula (IV)

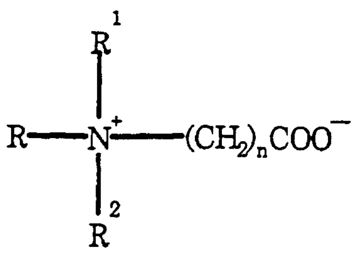

(IV)

where R, $R^1$ and $R^2$ are identical or different alkyl radicals having 1 - 10 carbon atoms, which may be branched and which may contain hydroxyl groups, in particular methyl radicals, and n = 1-3.

4. Betaine solutions prepared according to Claims 1 - 3, **characterized in that** 30 - 70% by weight of the $Li^+$, $Na^+$, $K^+$, $Mg^{2+}$, $Ca^{2+}$ salts with the organic acid are replaced by compounds of the general formula (IV)

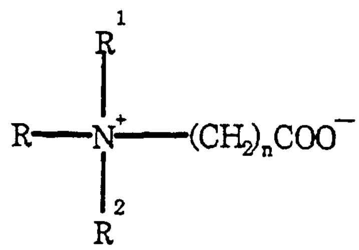

(IV)

where R, $R^1$ and $R^2$ are identical or different alkyl radicals having 1 - 10 carbon atoms, which may be branched and which may contain hydroxyl groups, in particular methyl radicals, and n = 1-3.

**Claims for the following Contracting States : BE, CH, LI, GB, NL**

1. Process for preparing highly concentrated flowable and pumpable aqueous solutions of betaines of the general formula III

$$R^3\text{-CONH-}(CH_2)_m\text{-}N^+R^4R^5(CH_2)_yCOO^- \quad \text{(III)}$$

where $R^3$ is the alkyl radical of a fatty acid, which may be branched, have multiple bonds and contain hydroxyl groups, $R^4$ and $R^5$ are identical or different alkyl radicals having 1 to 4 carbon atoms, m = 1 - 3 and y = 1, 2 or 3, by quaternization of compounds containing tertiary amine nitrogen, of the general formula I

$$R^3\text{-CONH-}(CH_2)_m\text{-}NR^4R^5 \quad \text{(I)}$$

where $R^3$, $R^4$, $R^5$ and m are as defined above, using ω-halocarboxylic acids by known processes, **characterized in that** before or during the quaternization reaction, 0.5 - 5% by weight, based on the total mixture, of at least one of the compounds

$$(Li^{1+})a(X^{n-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

where $X^{n-}$ is the radical of citric acid and n = 1 - 3, and in the molecule the value of $a^+ = b \cdot n^-$, are added to the fatty acid amide of the general formula I and to the alkali metal/alkaline earth metal salt of the ω-halocarboxylic acid.

2. Betaine solutions prepared according to Claim 1, **characterized in that** the salts present in the solution are 30 - 100 equivalent-% of salts of lithium, sodium, potassium, magnesium and/or calcium.

3. Betaine solutions prepared according to Claims 1 to 2, **characterized in that** they comprise

| | |
|---|---|
| >35% | by weight of compounds of the general formula (III) |
| 1.5-3% | by weight of at least one lithium, sodium, potassium, magnesium, calcium salt |
| to 100 | water. |

4. Process according to Claim 1, **characterized in that** 30 - 70% by weight of the $Li^+$, $Na^+$, $K^+$, $Mg^{2+}$, $Ca^{2+}$ salts of the organic acid are replaced by compounds of the general formula (IV),

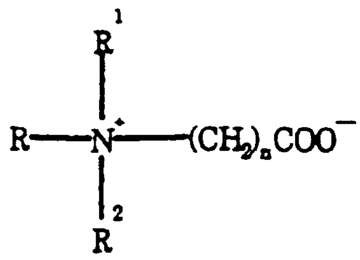

(IV)

where R, $R^1$ and $R^2$ are identical or different alkyl radicals having 1 - 10 carbon atoms, which may be branched and which may contain hydroxyl groups, in particular methyl radicals, and n = 1 - 3.

**5.** Betaine solutions according to Claims 2 - 3, **characterized in that** 30 - 70% by weight of the $Li^+$, $Na^+$, $K^+$, $Mg^{2+}$, $Ca^{2+}$ salts of the organic acid are replaced by compounds of the general formula (IV),

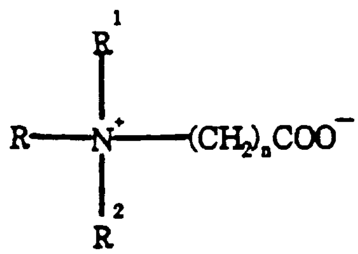

(IV)

where R, $R^1$ and $R^2$ are identical or different alkyl radicals having 1 - 10 carbon atoms, which may be branched and which may contain hydroxyl groups, in particular methyl radicals, and n = 1 - 3.

## Revendications

### Revendications pour les Etats contractants suivants : DE, ES, FR, IT

**1.** Procédé de préparation de solutions aqueuses fluides et susceptibles d'être pompées, très concentrées, de bétaïnes de formule générale (III) :

$$R^3\text{-CONH- }(CH_2)_m\text{-}N^+R^4R^5(CH_2)_yCOO^- \qquad \text{(III)}$$

où

$R^3$ représente le radical alcoyle d'un acide gras, qui peut être facultativement ramifié, peut contenir facultativement des liaisons multiples, facultativement des groupes hydroxyle;
$R^4$ et $R^5$ représentent des radicaux alcoyle identiques ou différents avec 1 à 4 atomes C;
m peut être 1 à 3, et
y peut être 1, 2, 3,

par quaternisation de composés contenant un azote d'amine tertiaire, de formule générale (I) :

$$R^3\text{-CONH- }(CH_2)_m\text{-}NR^4R^5 \qquad \text{(I)}$$

où $R^3$, $R^4$, $R^5$ et m ont la signification citée ci-dessus, avec un acide ω-halogénocarboxylique, par un procédé connu, **caractérisé en ce que** l'on ajoute à l'amide gras de formule générale (I) et au sel alcalin/alcalino-terreux de l'acide ω-halogénocarboxylique avant ou pendant la quaternisation, 0,5 à 5% en poids, sur base du mélange total, d'au moins l'un des composés :

$$(Li^{1+})_a(X^{n-})_b,\ (Na^{1+})_a(X^{n-})_b,\ (K^{1+})_a(X^{n-})_b,$$

$$(Mg^{2+})_a(X^{n-})_b,\ (Ca^{2+})_a(X^{n-})_b$$

où $X^{n-}$ est le radical de l'acide citrique, avec n = 1 à 3, et dans la molécule, la valeur de $a^+ = b\ .\ n^-$.

2. Procédé suivant la revendication 1, **caractérisé en ce que** les sels contenus dans la solution sont pour 30 à 100% en équivalents, des sels de lithium, sodium, potassium, magnésium et/ou calcium.

3. Procédé suivant la revendication 1, **caractérisé en ce que** 30 à 70% en poids des sels de $Li^+$, $Na^+$, $K^+$, $Mg^{2+}$, $Ca^{2+}$ de l'acide organique sont remplacés par des composés de formule générale (IV) :

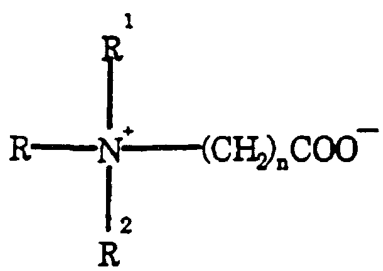

(IV)

où

R, $R^1$, $R^2$ sont des radicaux alcoyle identiques ou différents, facultativement ramifiés, contenant facultativement des groupes hydroxyle, avec 1 à 10 atomes C, en particulier un radical méthyle, et
n est 1 à 3.

4. Solutions de bétaïnes, préparées suivant les revendications 1 à 3, **caractérisées en ce que** 30 à 70% en poids des sels de $Li^+$, $Na^+$, $K^+$, $Mg^{2+}$, $Ca^{2+}$ de l'acide organique sont remplacés par des composés de formule générale (IV) :

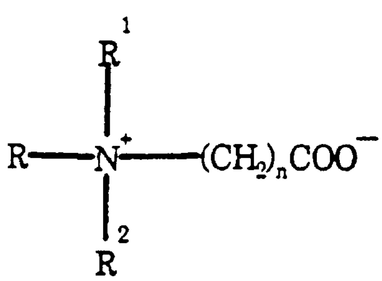

(IV)

où R, $R^1$, $R^2$ sont des radicaux alcoyle identiques ou différents, facultativement ramifiés, contenant facultativement des groupes hydroxyle, avec 1 à 10 atomes C, en particulier un radical méthyle, et
n est 1 à 3.

**Revendications pour les Etats contractants suivants : BE, CH, LI, GB, NL**

1. Procédé de préparation de solutions aqueuses fluides et susceptibles d'être pompées, très concentrées, de bétaïnes de formule générale (III):

$$R^3\text{-CONH-}(CH_2)_m\text{-}N^+R^4R^5(CH_2)_yCOO^- \qquad (III)$$

où $R^3$ représente la radical alcoyle d'un acide gras, qui peut être facultativement ramifié, peut contenir facultativement des liaisons multiples, facultativement des radicaux hydroxyle;

$R^4$ et $R^5$ représentent des radicaux alcoyle identiques ou différents avec 1 à 4 atomes C;
m peut être 1 à 3, et
y peut être 1, 2, 3,

par quaternisation de composés contenant un azote d'amine tertiaire, de formule générale (I):

$$R^3\text{-CONH-}(CH_2)_m\text{-}NR^4R^5 \qquad (I)$$

où $R^3$, $R^4$, $R^5$ et m ont la signification citée ci-dessus, avec un acide ω-halogénocarboxylique, par un procédé connu, **caractérisé en ce que** l'on ajoute à l'amide gras de formule générale (I) et au sel alcalin/alcalino-terreux de l'acide ω-halogénocarboxylique avant ou pendant la quaternisation, 0,5 à 5% en poids, sur base du mélange total, d'au moins l'un des composés :

$$(Li^{1+})_a(X^{n-})_b,\ (Na^{1+})_a(X^{n-})_b,\ (K^{1+})_a(X^{n-})_b,$$
$$(Mg^{2+})_a(X^{n-})_b,\ (Ca^{2+})_a(X^{n-})_b$$

où $X^{n-}$ est le radical de l'acide citrique, avec n = 1 à 3, et dans la molécule, la valeur de $a^+ = b.n^-$.

2. Solutions de bétaïnes, préparées suivant la revendication 1, **caractérisées en ce que** les sels contenus dans la solution sont pour 30 à 100% en équivalents, des sels de lithium, sodium, potassium, magnésium et/ou calcium.

3. Solutions de bétaïnes, préparées suivant les revendications 1 à 2, **caractérisées en ce qu'**elles contiennent :

| | |
|---|---|
| >35% | en poids de composés de formule générale (III); |
| 1,5 à 3% | en poids d'au-moins un sel de lithium, sodium, potassium, magnésium, calcium, et |
| pour faire 100 | d'eau. |

4. Procédé suivant la revendication 1, **caractérisé en ce que** 30 à 70% en poids des sels de $Li^+$, $Na^+$, $K^+$, $Mg^{2+}$, $Ca^{2+}$ de l'acide organique sont remplacés par des composés de formule générale (IV):

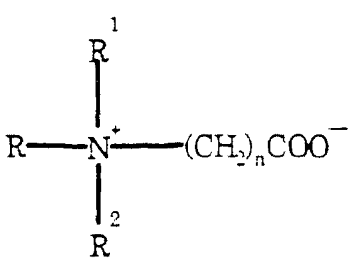

(IV)

où

$R$, $R^1$, $R^2$ sont des radicaux alcoyle identiques ou différents, facultativement ramifiés, contenant facultativement des radicaux hydroxyle, avec 1 à 10 atomes C, en particulier un radical méthyle, et
n est 1 à 3.

**5.** Solutions de bétaïnes suivant les revendications 2 et 3, **caractérisées en ce que** 30 à 70% en poids des sels de $Li^+$ $Na^+$, $K^+$, $Mg^{2+}$, $Ca^{2+}$ de l'acide organique sont remplacés par des composés de formule générale (IV):

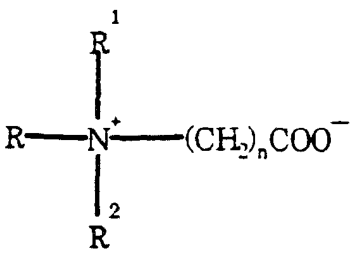

(IV)

où

$R$, $R^1$, $R^2$ sont des radicaux alcoyle identiques ou différents, facultativement ramifiés, contenant facultativement des radicaux hydroxyle, avec 1 à 10 atomes C, en particulier un radical méthyle, et
n est 1 à 3.